# FASCICULE DE BREVET EUROPEEN

(11) **EP 1 880 666 B1**
(45) Date de publication et mention de la délivrance du brevet: **01.07.2009**
(21) Numéro de dépôt: 06117653.3
(22) Date de dépôt: 21.07.2006
(51) Int. Cl.: A61B 5/024

(54) **Pulsomètre portable au poignet et procédé de commande associé**
Verfahren und Armbandgerät zur Pulsfrequenzermittlung
Method and wrist worn device for pulse rate detection

(43) Date de publication de la demande: 23.01.2008
(73) Titulaire: ETA SA Manufacture Horlogère Suisse, 2540 Grenchen (CH)
(72) Inventeur: Klopfenstein, François, 2800, Delémont (CH); Vetter, Rolf, 1116, Cottens (CH); Renevey, Philippe, 1005, Lausanne (CH); Neuman, Victor, 2036, Cormondrèche (CH); Verjus, Chritophe, 2000 Neuchâtel (CH)
(74) Mandataire: Ravenel, Thierry Gérard Louis

(56) Documents cités:
- WO-A-01/95796
- FR-A1- 2 669 819
- US-A- 4 063 551
- US-A- 4 706 072
- US-A1- 2003 065 269
- US-A1- 2004 236 227

## Description

L'invention concerne un pulsomètre portable au poignet et son procédé de commande.

L'invention concerne plus particulièrement un pulsomètre portable au poignet comportant un boîtier qui contient un dispositif électronique de mesure optique du pouls du porteur du pulsomètre et un circuit électronique pour le traitement des mesures en vue de calculer le pouls, un bracelet de serrage qui maintient le fond du boîtier plaqué contre le poignet, dans lequel le dispositif électronique de mesure optique comporte au moins une source lumineuse et plusieurs récepteurs de lumière qui sont agencés dans le fond du boîtier et qui sont orientés vers le poignet.

Un tel type de pulsomètre est décrit notamment dans le document US 2003/0065269. Dans ce document, le dispositif électronique de mesure optique comporte une diode émettrice qui est agencée au centre d'un carré défini par quatre photodiodes, de manière que chaque photodiode soit située à égale distance de la diode émettrice.

Bien que cette disposition de la diode et des photodiodes donne généralement de bons résultats, on constate parfois des problèmes de fiabilité des mesures de pouls, notamment en raison de différences physiologiques entre les différents porteurs, par exemple en matière de vascularisation du poignet. Ces problèmes de fiabilité des mesures peuvent aussi apparaître en raison de mauvais positionnements du boîtier sur le poignet.

L'invention vise notamment à remédier à ces inconvénients en proposant une solution simple et économique.

Dans ce but, l'invention propose un pulsomètre du type décrit précédemment, dans lequel le dispositif électronique de mesure optique comporte au moins deux sources lumineuses et au moins deux récepteurs, les sources lumineuses et les récepteurs étant agencés sous la forme d'une matrice comportant deux lignes, orientées chacune suivant une direction orthogonale à la direction du poignet, et au moins deux colonnes, orientées parallèlement à la direction du poignet caractérisé, en ce que chaque ligne de la matrice contient alternativement une source lumineuse et un récepteur, et chaque colonne de la matrice contient une source lumineuse et un récepteur.

Grâce à l'agencement selon l'invention, on a constaté une augmentation de la fiabilité des mesures malgré le fait que les récepteurs ne sont pas répartis de manière symétrique autour de chaque source lumineuse. En particulier, le décalage des colonnes de sources lumineuses et de récepteurs selon une direction orthogonale à la direction du poignet permet de couvrir une grande variété de caractéristiques physiologies différentes parmi les porteurs, ce qui permet de compenser les différences de vascularisation.

L'agencement selon l'invention permet aussi de détecter plus facilement un mauvais positionnement du boîtier du pulsomètre sur le poignet, ce qui permet au porteur de détecter plus facilement l'origine des erreurs de mesure du pouls.

De préférence, la distance entre chaque source lumineuse et le récepteur adjacent dans une ligne de la matrice est sensiblement égale à la distance entre chaque source lumineuse et le récepteur adjacent dans une colonne de la matrice. Un tel agencement facilite le traitement du signal produit par chaque récepteur en homogénéisant l'intensité des signaux.

Selon un mode de réalisation avantageux, la matrice comporte trois colonnes, et la première ligne contient une source lumineuse entourée par deux récepteurs, et la seconde ligne contient un récepteur entouré par deux sources lumineuses. Cette disposition offre un compromis particulièrement efficace en couvrant un plus grand nombre de physiologies des porteurs différentes tout en permettant de réaliser un dispositif électronique de mesure optique compact et économique. En particulier, cette disposition minimise la surface occupée sur le fond du boîtier, notamment dans la direction du poignet, par rapport à un dispositif selon l'art antérieur.

Dans le cadre de la présente invention, il a été constaté de manière inattendue que l'agencement non symétrique des récepteurs par rapport aux sources lumineuses n'a pas diminué la fiabilité des signaux produits par les récepteurs. Au contraire, cet agencement permet d'obtenir au moins un signal de réception fiable dans la majorité des cas, quel que soit la morphologie du porteur et ses caractéristiques de vascularisation.

De préférence, chaque source lumineuse est constituée par une diode qui émet de la lumière dans le domaine de l'infrarouge, et chaque récepteur est constitué par une photodiode. Ce système de mesure optique est celui qui offre les meilleurs résultats en matière de fiabilité et de qualité des mesures, tout en étant économique et simple de mise en oeuvre.

Selon un mode de réalisation avantageux, le circuit électronique comprend une unité de calcul du pouls qui calcule une valeur de pouls correspondant respectivement à chaque signal de réception produit par un récepteur et à un signal virtuel obtenu par une unité de calcul d'un signal virtuel qui correspond à une addition des signaux de réception produits par chacun des récepteurs. Une unité de sélection détermine une valeur de pouls optimale parmi les valeurs de pouls obtenues par l'unité de calcul du pouls. Cette solution augmente encore la diversité des signaux exploitables et permet de compenser les erreurs de mesures entre les différents récepteurs.

Avantageusement, le circuit électronique comprend une unité de calcul d'un indice de fiabilité des mesures qui est fonction des valeurs de pouls obtenues par l'unité de calcul du pouls. Cet indice de fiabilité permet d'exploiter au mieux la diversité des signaux produits par les récepteurs en permettant d'éviter d'afficher des valeurs de pouls irréalistes et en permettant d'informer l'utilisateur sur la qualité des mesures effectuées. De plus, lorsque cet indice de fiabilité atteint une valeur déterminée, le circuit électronique peut détecter un mauvais positionnement du boîtier sur le poignet ou un état non porté du pulsomètre.

L'invention propose aussi un procédé de commande d'un pulsomètre selon la revendication 1 comportant une étape de mesure au cours de laquelle chaque source lumineuse émet un faisceau lumineux et chaque récepteur produit un signal de réception en fonction de la lumière reçue, et une étape de calcul du pouls au cours de laquelle une valeur de pouls est calculée à partir du signal de réception produit par chaque récepteur au cours de l'étape de mesure, dans lequel une étape de calcul d'un signal virtuel correspondant à une addition des signaux de réception produits par chacun des récepteurs est intercalée entre l'étape de mesure et l'étape de calcul du pouls, dans lequel une valeur de pouls est calculée à partir du signal virtuel au cours de l'étape de calcul du pouls, et dans lequel, au cours de l'étape de sélection, la valeur de pouls optimale est sélectionnée parmi les valeurs de pouls obtenues à l'étape de calcul du pouls.

Selon d'autres caractéristiques de ce procédé :
- l'étape de calcul du pouls est suivie par une étape de calcul d'un indice de fiabilité des mesures au cours de laquelle une comparaison est effectuée entre les valeurs de pouls obtenues à l'étape de calcul du pouls;
- l'étape de calcul de l'indice de fiabilité des mesures est suivie par une étape de détection de l'état de positionnement du boîtier au cours de laquelle, en fonction de la valeur de l'indice de fiabilité, est déterminé si le pulsomètre est porté ou si le boîtier est mal positionné sur le poignet.

D'autres caractéristiques et avantages de la présente invention apparaîtront plus clairement à la lecture de la description détaillée qui suit, faite en référence aux dessins annexés donnés à titre d'exemple non limitatifs et dans lesquels :
- la figure 1 est une vue de dessus qui représente schématiquement le pulsomètre selon l'invention porté au poignet d'un porteur;
- la figure 2 est une vue de dessous qui représente schématiquement le fond du boîtier du pulsomètre de la figure 1 et son dispositif électronique de mesure optique du pouls;
- la figure 3 est une vue en coupe selon le plan 3-3 qui représente schématiquement le pulsomètre de la figure 1 et le faisceau lumineux émis par une source lumineuse du dispositif électronique de mesure optique;
- la figure 4 est un schéma fonctionnel qui représente le pulsomètre de la figure 1 et le circuit électronique qui l'équipe.

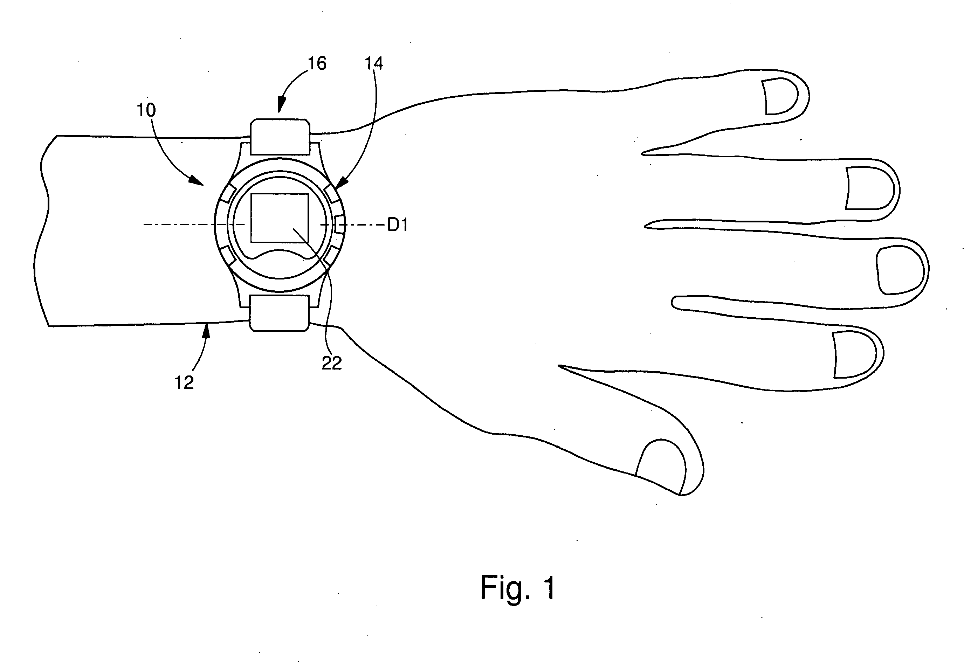

Sur les figures 1 à 4, on a représenté un pulsomètre 10 portable au poignet 12 d'un porteur. Le pulsomètre 10 comporte un boîtier 14 attaché au poignet 12 par l'intermédiaire d'un bracelet 16 de serrage.

Dans la suite de la description, on désignera par direction D1 du poignet 12 la direction générale de l'avant-bras associé au poignet 12 du porteur.

Le boîtier 14 contient un dispositif électronique de mesure optique 18 du pouls du porteur et un circuit électronique 20 pour le traitement des mesures en vue de calculer le pouls P du porteur et de l'afficher au moyen d'un dispositif d'affichage 22 tel qu'un écran à cristaux liquides.

Le dispositif électronique de mesure optique 18 est agencé dans le fond 24 du boîtier 14, du côté opposé au dispositif d'affichage 22. Le bracelet 16 est prévu pour maintenir le fond 24 du boîtier 14 plaqué contre le poignet 12, de manière à optimiser le fonctionnement du dispositif électronique de mesure optique 18.

Conformément aux enseignements de l'invention, le dispositif électronique de mesure optique 18 comporte au moins deux sources lumineuses E1, E2, E3 et au moins deux récepteurs R1, R2, R3 de lumière qui sont orientés vers le poignet 12 du porteur et qui sont agencés sous la forme d'une matrice comportant deux lignes L1, L2, orientées suivant une direction orthogonale à la direction D1 du poignet 12, et au moins deux colonnes C1, C2, C3, orientées parallèlement à la direction D1 du poignet. De plus, chaque ligne L1, L2 de la matrice contient alternativement une source lumineuse E1, E2, E3 et un récepteur R1, R2, R3, et chaque colonne C1, C2, C3 de la matrice contient une source lumineuse E1, E2, E3 et un récepteur R1, R2, R3.

Selon un mode de réalisation préféré, qui est représenté sur les figures, le dispositif électronique de mesure optique 18 comporte trois sources lumineuses E1, E2, E3, constituées par trois diodes émettant dans le domaine de l'infrarouge, et trois récepteurs R1, R2, R3, constitués par trois photodiodes prévues pour produire chacune un signal de réception SR1, SR2, SR3 qui est fonction de la quantité de lumière reçue.

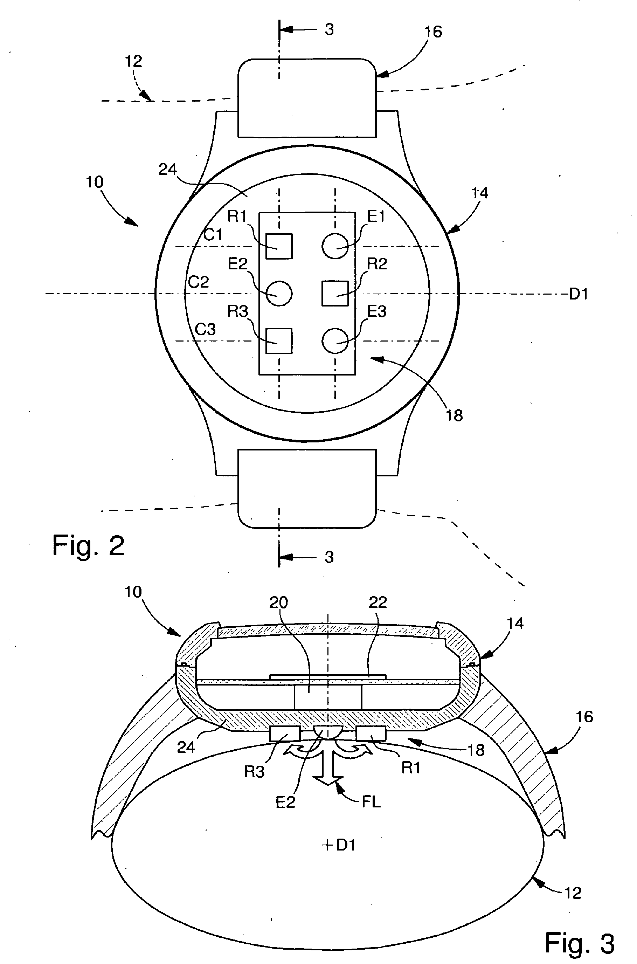

La matrice comporte donc ici trois colonnes C1, C2, C3 avec, en considérant la figure 2, une première colonne C1 comportant un premier récepteur R1 dans la première ligne L1 et une première source lumineuse E1 dans la seconde ligne L2, une deuxième colonne C2 comportant une deuxième source lumineuse E2 dans la première ligne L1 et un deuxième récepteur R2 dans la seconde ligne L2, et une troisième colonne C3 comportant un troisième récepteur R3 dans la première ligne L1 et une troisième source lumineuse E3 dans la seconde ligne L2.

Dans la première ligne L1, la deuxième source lumineuse E2 est donc sensiblement alignée avec le premier et le troisième récepteurs R1, R3. Dans la seconde ligne L2, la première et la troisième sources lumineuses E1, E3 sont donc sensiblement alignées avec le deuxième récepteur R2.

De préférence, la distance entre chaque source lumineuse E1, E2, E3 et le récepteur R1, R2, R3 adjacent dans une ligne L1, L2 de la matrice est sensiblement égale à la distance entre chaque source lumineuse E1, E2, E3 et le récepteur R1, R2, R3 adjacent dans une colonne C1, C2 de la matrice.

Selon le mode de réalisation représenté sur les figures, les lignes L1, L2 sont rectilignes mais elles pourraient aussi être courbées et décrire deux arcs de cercles sensiblement parallèles, on sécants.

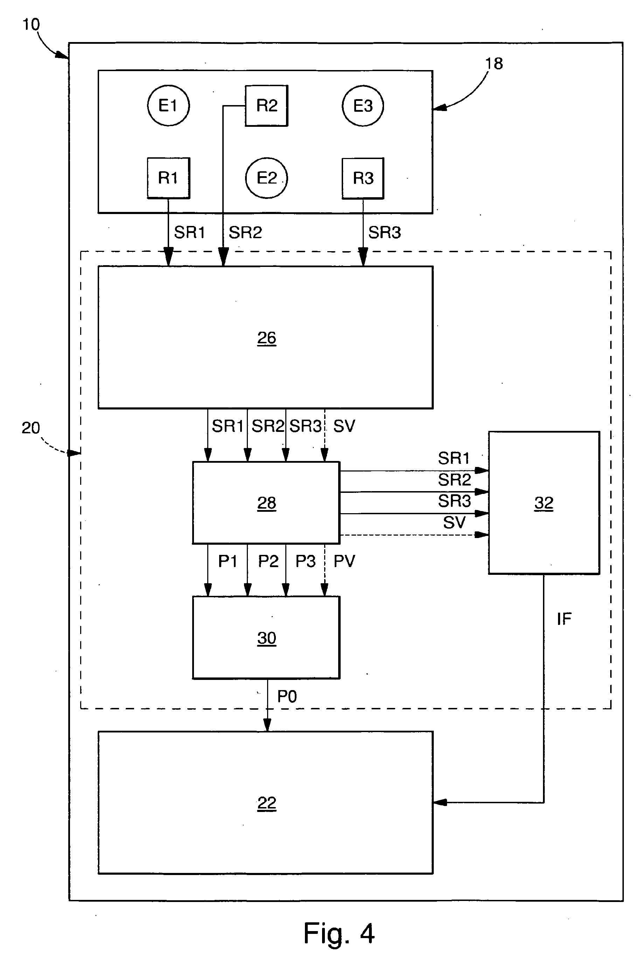

Sur la figure 4, on a représenté plus en détail le circuit électronique 20 du pulsomètre 10 selon l'invention.

Selon le mode de réalisation préféré, le circuit électronique 20 comprend une première unité de calcul 26 qui détermine, à partir des signaux de réception SR1, SR2, SR3 produits par les trois récepteurs R1, R2, R3 lors de chaque mesure de pouls, un signal de réception virtuel SV associé. Le signal de réception virtuel SV est de préférence constitué par une addition des trois signaux de réception SR1, SR2, SR3.

Le circuit électronique 20 comporte une deuxième unité de calcul 28 qui détermine, à partir des signaux de réception SR1, SR2, SR3 produits par les récepteurs R1, R2, R3 et à partir du signal virtuel SV, les valeurs de pouls P1, P2, P3, PV correspondantes. La deuxième unité de calcul 28 est prévue pour traiter les signaux SR1, SR2, SR3, SV en éliminant le bruit, par exemple à l'aide de filtres (non représentés), ce bruit étant dû principalement aux micro-mouvements du boîtier 14 par rapport au poignet 12 du porteur.

Le circuit électronique 20 comporte une unité de sélection 30 qui sélectionne, parmi les valeurs de pouls P1, P2, P3, PV obtenues par la deuxième unité de calcul 28, une valeur de pouls optimale PO. Cette valeur de pouls optimale PO est sélectionnée sur la base de critères définis par conception, par exemple la valeur de pouls P1, P2, P3, PV possédant la plus petite variance est sélectionnée comme valeur optimale PO.

La valeur de pouls optimale PO sélectionnée par l'unité de sélection 30 est transmise au dispositif d'affichage 22 pour permettre au porteur de la visualiser.

Bien entendu, à partir de la valeur de pouls optimale PO, d'autres paramètres liés à la valeur du pouls PO peuvent aussi être calculés et affichés, par exemple la quantité de calories consommées, ou d'autres informations liées à l'historique des mesures de pouls.

Selon le mode de réalisation préféré, le circuit électronique 20 comprend une troisième unité de calcul 32 qui détermine la valeur d'un indice de fiabilité IF des mesures de pouls à partir des valeurs de pouls P1, P2, P3, PV obtenues par la deuxième unité de calcul 28. La troisième unité de calcul 32 affecte une valeur d'indice de fiabilité IF qui est, par exemple, fonction de la fréquence des signaux correspondant aux valeurs de pouls P1, P2, P3, PV, de la corrélation entre ces signaux, de l'amplitude de ces signaux, et de l'historique des valeurs de pouls P1, P2, P3, PV.

Concernant la fréquence, il faut que les signaux soient compris dans une bande spectrale déterminée.

Lorsque plusieurs signaux sont corrélés, c'est-à-dire que plusieurs signaux donnent la même information, notamment en ayant des variations similaires au même moment, cela indique que cette information est fiable puisque la diversité spatiale des récepteurs R1, R2, R3 fait qu'ils sont influencés différemment lorsque le boîtier 14 est mal positionné.

L'amplitude de ces signaux doit être comprise dans des limites déterminées, de sorte qu'une amplitude trop importante permet de détecter un problème de fiabilité de la mesure dû à des mouvements du porteur.

La détermination de l'indice de fiabilité IF exploite par exemple le résultat des calculs de variance et de dispersion des valeurs de pouls P1, P2, P3, PV.

Selon une variante de réalisation de l'invention, la troisième unité de calcul 32 peut aussi utiliser les signaux de réception SR1, SR2, SR3 et le signal virtuel SV pour déterminer la valeur de l'indice de fiabilité IF.

La troisième unité de calcul 32 peut transmettre la valeur de l'indice de fiabilité IF au dispositif d'affichage 22 pour permettre au porteur de connaître la fiabilité des valeurs de pouls P qui sont affichées. En fonction de la valeur de l'indice de fiabilité IF, lorsque celle-ci est représentative d'une fiabilité insuffisante des mesures, le circuit électronique 20 peut aussi commander la suspension de l'affichage du pouls P, de manière à ne pas afficher de valeurs de pouls P erronées.

Avantageusement, en fonction de la valeur de l'indice de fiabilité IF, le circuit électronique 20 peut détecter un mauvais positionnement du boîtier 14 sur le poignet 12, ce qui se traduit par une plus grande quantité de lumière ambiante qui atteint au moins l'un des récepteurs R1, R2, R3.

Dans le cadre de la présente invention, après de nombreuses expérimentations et essais, il a été constaté que la configuration selon le mode de réalisation préféré avec trois sources lumineuses E1, E2, E3 et trois récepteurs R1, R2, R3 est celle qui offre le meilleur compromis pour l'amélioration de la fiabilité des mesures de pouls en couvrant un nombre important de porteurs avec des caractéristiques physiologiques différentes, tout en limitant l'encombrement et la complexité du dispositif électronique de mesure optique 18. De plus, cette configuration est celle qui donne les meilleurs résultats pour détecter des problèmes de mauvais positionnement du boîtier 14 sur le poignet 12.

On note que, dans le mode de réalisation préféré, le deuxième récepteur R2, qui est situé au centre de la seconde ligne L2 et qui est donc entouré par trois sources lumineuses E1, E2, E3, reçoit de la lumière provenant de ces trois sources lumineuses E1, E2, E3, alors que chacun des deux autres récepteurs R1, R3 reçoivent essentiellement de la lumière provenant des deux sources lumineuses adjacentes, respectivement E1, E2 et E2, E3. Pour compenser ce déséquilibre dans la réception de lumière, il est prévu de diminuer le gain dans le canal du circuit électronique analogique traitant le signal de réception SR2 produit par le deuxième récepteur R2. Alternativement, le susdit déséquilibre du signal SR2 peut être corrigé numériquement par l'unité de calcul 26.

On décrit maintenant le procédé de commande du pulsomètre selon l'invention.

Ce procédé comporte une étape de mesure, mise en oeuvre par le dispositif électronique de mesure optique 18, au cours de laquelle chaque source lumineuse E1, E2, E3 émet un faisceau lumineux FL dirigé vers le poignet 12 du porteur. Ce faisceau lumineux FL se propage dans le poignet 12 et une partie de ce faisceau lumineux FL est rétro-diffusée et détectée par les récepteurs R1, R2, R3. En fonction de la quantité de lumière reçue, chaque récepteur produit un signal de réception SR1, SR2, SR3 qui permet la mesure des pulsations cardiaques du porteur en détectant des variations périodiques de l'énergie lumineuse absorbée par les tissus du porteur.

Le principe de cette étape de mesure du pouls P est décrit notamment dans le document US 2003/0065269 auquel on pourra se reporter pour plus de détails, en particulier dans le préambule de la description de ce document.

Conformément aux enseignements de l'invention, l'étape de mesure du pouls P est suivie par une étape de calcul du signal virtuel SV, mise en oeuvre par la première unité de calcul 26, au cours de laquelle est produit le signal virtuel SV correspondant à une addition des signaux de réception SR1, SR2, SR3.

L'étape de calcul du signal virtuel SV est suivie par une étape de calcul du pouls P1, P2, P3, PV, mise en oeuvre par la deuxième unité de calcul 28, au cours de laquelle les signaux de réception SR1, SR2, SR3 et le signal virtuel SV sont traités de manière à déterminer les valeurs de pouls P1, P2, P3, PV correspondantes.

L'étape de calcul du pouls est suivie par une étape de sélection, mise en oeuvre par l'unité de sélection 30, au cours de laquelle la valeur de pouls optimale PO est sélectionnée parmi les valeurs de pouls P1, P2, P3, PV obtenues au cours de l'étape de calcul du pouls. C'est cette valeur de pouls optimale PO qui est prévue pour être affichée.

De préférence, l'étape de calcul du pouls est suivie par une étape de calcul de l'indice de fiabilité IF des mesures, mise en oeuvre par la troisième unité de calcul 32, au cours de laquelle les valeurs de pouls P1, P2, P3 correspondant aux signaux de réception SR1, SR2, SR3 et la valeur de pouls PV correspondant au signal virtuel SV sont comparées entre elles de manière à déterminer la valeur de l'indice de fiabilité IF représentatif de la confiance qui peut être placée dans les mesures effectuées et dans la valeur de pouls optimale PO obtenue par l'unité de sélection 30.

On note que l'étape de calcul de l'indice de fiabilité IF peut prévoir de comparer les valeurs de pouls P1, P2, P3, PV obtenues par l'étape de calcul du pouls en cours aux valeurs antérieures qui ont été obtenues lors des étapes de calcul correspondant aux mesures précédentes et qui ont été mémorisés, ce qui permet de déterminer si l'évolution de la valeur du pouls PO au cours du temps est réaliste.

L'indice de fiabilité IF peut être calculé en tenant compte des valeurs d'amplitude des composantes continue et alternative de chaque valeur de pouls P1, P2, P3, PV dues à la lumière ambiante, et des valeurs d'amplitude des composantes continue et alternative de chaque signal P1, P2, P3, PV dues à la lumière émise par les sources E1, E2, E3, et en tenant compte de valeurs dérivées de ces signaux P1, P2, P3, PV, comme par exemple la variance ou le spectre de fréquences. En tenant compte de ces paramètres, il est ainsi possible de distinguer le cas où le pulsomètre 10 est mal positionné sur le poignet 12 du cas où le pulsomètre 10 est non porté, par exemple lorsqu'il est posé sur une table.

Avantageusement, l'étape de calcul de l'indice de fiabilité IF est suivie par une étape de détection d'un mauvais positionnement du boîtier 14 sur le poignet 12 ou d'un état non porté du pulsomètre 10 au cours de laquelle, en fonction de la valeur de l'indice de fiabilité IF, le circuit électronique 20 signale au porteur le mauvais positionnement, par exemple au moyen du dispositif d'affichage 22, ou le circuit électronique 20 interrompt l'affichage du pouls dans le cas d'une détection d'un état non porté.

## Revendications

1. Pulsomètre (10) portable au poignet (12) comportant un boîtier (14) qui contient un dispositif électronique de mesure optique (18) du pouls du porteur du pulsomètre (10) et un circuit électronique (20) pour le traitement des mesures en vue de calculer le pouls (P), un bracelet (16) de serrage qui maintient le fond (24) du boîtier (14) plaqué contre le poignet (12), dans lequel le dispositif électronique de mesure optique (18) comporte au moins deux sources lumineuses (E1, E2, E3) et au moins deux récepteurs (R1, R2, R3) de lumière qui sont agencés dans le fond (24) du boîtier (14) et qui sont orientés vers le poignet (12), les sources lumineuses (E1, E2, E3) et les récepteurs (R1, R2, R3) étant agencés sous la forme d'une matrice comportant deux lignes (L1, L2), orientées chacune suivant une direction orthogonale à la direction (D1) du poignet (12), et au moins deux colonnes (C1, C2, C3), orientées parallèlement à la direction (D1) du poignet (12), **caractérisé en ce que** chaque ligne (L1, L2) de la matrice contient alternativement une source lumineuse (E1, E2, E3) et un récepteur (R1, R2, R3), et chaque colonne (C1, C2, C3) de la matrice contient alternativement une source lumineuse (E1, E2, E3) et un récepteur (R1, R2, R3)

2. Pulsomètre (10) selon la revendication précédente, dans lequel la distance entre chaque source lumineuse (E1, E2, E3) et le récepteur (R1, R2, R3) adjacent dans une ligne (L1, L2) de la matrice est sensiblement égale à la distance entre chaque source lumineuse (E1, E2, E3) et le récepteur (R1, R2, R3) adjacent dans une colonne (C1, C2, C3) de la matrice.

3. Pulsomètre (10) selon la revendication 1 ou 2, dans lequel la matrice comporte trois colonnes (C1, C2, C3), et dans lequel la première ligne (L1) contient une source lumineuse (E2) entourée par deux récepteurs (R1, R3), et la seconde ligne (L2) contient un récepteur (R2) entouré par deux sources lumineuses (E1, E3).

4. Pulsomètre (10) selon l'une quelconque des revendications précédentes, dans lequel chaque source lumineuse (E1, E2, E3) est constituée par une diode qui émet de la lumière dans le domaine de l'infrarouge, et chaque récepteur (R1, R2, R3) est constitué par une photodiode.

5. Pulsomètre (10) selon l'une quelconque des revendications précédente, dans lequel le circuit électronique (20) comprend une unité de calcul du pouls (28) qui calcule une valeur de pouls (P1, P2, P3) correspondant respectivement à chaque signal de réception (SR1, SR2, SR3) produit par un récepteur (R1, R2, R3), et une unité de sélection (30) qui détermine une valeur de pouls optimale (PO) parmi les valeurs de pouls (P1, P2, P3) obtenues par l'unité de calcul du pouls (28).

6. Pulsomètre (10) selon l'une quelconque des revendications 1 à 4, dans lequel le circuit électronique (20) comprend une unité de calcul (26) d'un signal virtuel (SV) correspondant à une addition des signaux de réception (SR1, SR2, SR3) produits par chacun des récepteurs (R1, R2, R3), une unité de calcul du pouls (28) qui calcule une valeur de pouls (P1, P2, P3, PV) correspondant respectivement à chaque signal de réception (SR1, SR2, SR3) produit par un récepteur (R1, R2, R3) et au signal virtuel (SV), et une unité de sélection (30) qui détermine une valeur de pouls optimale (PO) parmi les valeurs de pouls (P1, P2, P3, PV) obtenues par l'unité de calcul du pouls (28).

7. Pulsomètre (10) selon la revendication 5 ou 6, dans lequel le circuit électronique (20) comprend une unité de calcul (32) d'un indice de fiabilité (IF) des mesures qui est fonction des valeurs de pouls (P1, P2, P3, PV) obtenues par l'unité de calcul du pouls (28).

8. Procédé de commande d'un pulsomètre (10) selon l'une quelconque des revendications précédentes, comportant une étape de mesure au cours de laquelle chaque source lumineuse (E1, E2, E3) émet un faisceau lumineux (FL) et chaque récepteur (R1, R2, R3) produit un signal de réception (SR1, SR2, SR3) en fonction de la lumière reçue, et une étape de calcul du pouls au cours de laquelle une valeur de pouls (P1, P2, P3) est calculée à partir du signal de réception (SR1, SR2, SR3) produit par chaque récepteur (R1, R2, R3) au cours de l'étape de mesure, dans lequel l'étape de calcul du pouls (P1, P2, P3) est suivie par une étape de sélection au cours de laquelle une valeur de pouls optimale (PO) est sélectionnée parmi les valeurs de pouls (P1, P2, P3) obtenues à l'étape de calcul du pouls.

9. Procédé de commande d'un pulsomètre (10) selon l'une quelconque des revendications 1 à 7, comportant une étape de mesure au cours de laquelle chaque source lumineuse (E1, E2, E3) émet un faisceau lumineux (FL) et chaque récepteur (R1, R2, R3) produit un signal de réception (SR1, SR2, SR3) en fonction de la lumière reçue, et une étape de calcul du pouls au cours de laquelle une valeur de pouls (P1, P2, P3) est calculée à partir du signal de réception (SR1, SR2, SR3) produit par chaque récepteur (R1, R2, R3) au cours de l'étape de mesure, dans lequel une étape de calcul d'un signal virtuel (SV) correspondant à une addition des signaux de réception (SR1, SR2, SR3) produits par chacun des récepteurs (R1, R2, R3) est intercalée entre l'étape de mesure et l'étape de calcul du pouls, dans lequel une valeur de pouls (PV) est calculée à partir du signal virtuel (SV) au cours de l'étape de calcul du pouls, et dans lequel, au cours de l'étape de sélection, la valeur de pouls optimale (PO) est sélectionnée parmi les valeurs de pouls (P1, P2, P3, PV) obtenues à l'étape de calcul du pouls.

10. Procédé de commande selon la revendication 8 ou 9, dans lequel l'étape de calcul du pouls est suivie par une étape de calcul d'un indice de fiabilité (IF) des mesures au cours de laquelle une comparaison est effectuée entre les valeurs de pouls (P1, P2, P3, PV) obtenues à l'étape de calcul du pouls.

11. Procédé de commande selon la revendication précédente, dans lequel l'étape de calcul de l'indice de fiabilité (IF) des mesures est suivie par une étape de détection de l'état de positionnement du boîtier (14) au cours de laquelle, en fonction de la valeur de l'indice de fiabilité (IF), est déterminé si le pulsomètre (10) est porté ou si le boîtier (14) est mal positionné sur le poignet (12).

## Claims

1. Pulsometer (10) worn on the wrist (12) including a case (14), which contains an electronic optical device (18) for measuring the pulse of the wearer of the pulsometer (10) and an electronic circuit (20) for processing the measurements in order to calculate the pulse (P), a tightening wristband (16), which holds the back cover (24) of the case (14) pressed against the wrist (12), wherein the electronic optical measuring device (18) includes at least two light sources (E1, E2, E3) and at least two light receivers (R1, R2, R3) which are arranged in the back cover (24) of the case (14) and which are oriented towards the wrist (12),
wherein the light sources (E1, E2, E3) and the receivers (R1, R2, R3) are arranged in the form of a matrix including two lines (L1, L2) each oriented along an orthogonal direction to the direction (D1) of the wrist (12), and at least two columns (C1, C2, C3) oriented parallel to the direction (D1) of the wrist (12), **characterized in that** each line (L1, L2) of the matrix alternately contains a light source (E1, E2, E3) and a receiver (R1, R2, R3) , and each column (C1, C2, C3) of the matrix alternately contains a light source (E1, E2, E3) and a receiver (R1, R2, R3).

2. Pulsometer (10) according to the preceding claim , wherein the distance between each light source (E1, E2, E3) and the adjacent receiver (R1, R2, R3) in one line (L1. L2) of the matrix is substantially equal to the distance between each light source (E1, E2, E3) and the adjacent receiver (R1, R2, R3) in one column (C1, C2, C3) of the matrix.

3. Pulsometer (10) according to claim 1 or 2, wherein the matrix includes three columns (C1, C2, C3) and wherein the first line (L1) contains one light source (E2) surrounded by two receivers (R1, R3), and the second line (L2) contains one receiver (R2) surrounded by two light sources (E1, E3).

4. Pulsometer (10) according to any of the preceding claims, wherein each light source (E1, E2, E3) is formed by a diode which emits light in the infrared range, and each receiver (R1, R2, R3) is formed by a photodiode.

5. Pulsometer (10) according to any of the preceding claims, wherein the electronic circuit (20) includes a pulse calculating unit (28) which calculates a pulse value (P1, P2, P3) corresponding respectively to each reception signal (SR1, SR2, SR3) produced by a receiver (R1, R2, R3) and a selection unit (30) which determines an optimum pulse value (PO) from among the pulse values (P1, P2, P3) obtained by the pulse calculating unit (28).

6. Pulsometer (10) according to any of claims 1 to 4, wherein the electronic circuit (20) includes a unit (26) for calculating a virtual signal (SV) corresponding to an addition of the reception signals (SR1, SR2, SR3) produced by each of the receivers (R1, R2, R3), a pulse calculating unit (28) which calculates a pulse value (P1, P2, P3, PV) corresponding respectively to each reception signal (SR1, SR2, SR3) produced by one receiver (R1, R2, R3) and to the virtual signal (SV), and a selection unit (30) which determines an optimum pulse value (PO) from among the pulse values (PV) obtained by the pulse calculating unit (28).

7. Pulsometer (10) according to claim 5 or 6, wherein the electronic circuit (20) includes a unit (32) for calculating a measurement reliability index (IF) which is a function of the pulse values (P1, P2, P3) obtained by the pulse calculating unit (28).

8. Control method for a pulsometer (10) according to any of the preceding claims, including a measurement step during which each light source (E1, E2, E3) emits a light beam (FL) and each receiver (R1, R2, R3) produces a reception signal (SR1, SR2, SR3) as a function of the light received, and a pulse calculating step during which a pulse value (P1, P2, P3) is calculated from the reception signal (SR1, SR2, SR3) produced by each receiver (R1, R2, R3) during the measuring step, wherein the pulse calculating step (P1, P2, P3) is followed by a selection step during which an optimum pulse value (PO) is selected from among the pulse values (P1, P2, P3) obtained in the pulse calculating step.

9. Control method for a pulsometer (10) according to any of claims 1 to 7, including a measuring step during which each light source (E1, E2, E3) emits a light beam (FL) and each receiver (R1, R2, R3) produces a reception signal (SR1, SR2, SR3) as a function of the light received, and a pulse calculating step during which a pulse value (P1, P2, P3) is calculated from the reception signal (SR1, SR2, SR3) produced by each receiver (R1, R2, R3) during the measuring step , wherein a step of calculating a virtual signal (SV) corresponding to an addition of the reception signals (SR1, SR2, SR3) produced by each of the receivers (R1, R2, R3) is inserted between the measuring step and the pulse calculating step, wherein a pulse value (PV) is calculated from the virtual signal (SV), during the pulse calculating step, and wherein, during the selection step, the optimum pulse value (PO) is selected from the pulse values (P1, P2, P3) obtained in the pulse calculating step.

10. Control method according to claim 8 or 9, wherein the pulse calculating step is followed by a step of calculating a measurement reliability index (IF) during which a comparison is made between the pulse values (P1, P2, P3, PV) obtained in the pulse calculating step.

11. Control method according to the preceding claim, wherein the measurement reliability index (IF) calculating step is followed by a step of detecting the positioning step state of the case (14) during which, as a function of the reliability index value (IF), it is determined whether the pulsometer (10) is being worn or whether the case (14) is poorly positioned on the wrist (12).

## Patentansprüche

1. Am Handgelenk (12) tragbarer Pulsmesser (10) umfassend ein Gehäuse (14), welches eine elektronische Vorrichtung (18) zum optischen Messen des Pulses des Trägers des Pulsmessers (10) und einen elektronischen Schaltkreis (20) zum Verarbeiten der Messungen zum Berechnen des Pulses (P) umfasst, ein Klemmarmband (16), welches den Boden (24) des Gehäuses (14) gegen das Handgelenk (12) gepresst hält, wobei die elektronische Vorrichtung (18) zum optischen Messen wenigstens zwei Lichtquellen (E1, E2, E3) und wenigsten zwei Lichtempfänger (R1, R2, R3) umfasst, die in dem Boden (24) des Gehäuses (14) angeordnet sind, und die in Richtung des Handgelenks (12) ausgerichtet sind,
wobei die Lichtquellen (E1, E2, E3) und die Empfänger (R1, R2, R3) in Form einer Matrix angeordnet sind, die zwei Reihen (L1, L2) umfasst, die jeweils entlang einer Richtung senkrecht zu der Richtung (D1) des Handgelenkes (12) ausgerichtet sind, und wenigstens zwei parallel zu der Richtung (D1) des Handgelenkes (12) ausgerichtete Spalten (C1, C2, C3), **dadurch gekennzeichnet, dass** jede Reihe (L1, L2) der Matrix abwechselnd eine Lichtquelle (E1, E2, E3) und einen Empfänger (R1, R2, R3), und jede Spalte (G1, C2, C3) der Matrix jeweils abwechselnd eine Lichtquelle (E1, E2, E3) und einen Empfänger (R1, R2, R3) enthält.

2. Pulsmesser (10) gemäss dem vorstehenden Anspruch, wobei der Abstand zwischen jeder Lichtquelle (E1, E2, E3) und dem in einer Reihe (L1, L2) der Matrix daneben liegenden Empfänger (R1, R2, R3), im Wesentlichen gleich dem Abstand zwischen jeder Lichtquelle (E1, E2, E3) und dem in einer Spalte (C1, C2, C3) der Matrix daneben liegenden Empfänger (R1, R2, R3) entspricht.

3. Pulsmesser (10) gemäss Anspruch 1 oder 2, wobei die Matrix drei Spalten (C1, C2, C3) umfasst, und wobei die erste Reihe (L1) eine Lichtquelle (E2) umfasst, die von zwei Empfängern (R1, R3) eingerahmt ist, und die zweite Reihe (L2) einen Empfänger (R2) umfasst, der von zwei Lichtquellen (E1, E3) eingerahmt ist.

4. Pulsmesser (10) nach einem der vorstehenden Ansprüche, **dadurch gekennzeichnet, dass** jede Lichtquelle (E1, E2, E3) von einer Diode gebildet ist, die Licht in Infrarotbereich aussendet, und dass jeder Empfänger (R1, R2, R3) von einer Fotodiode gebildet ist.

5. Pulsmesser (10) gemäss einem der vorstehenden Ansprüche, wobei der elektronische Schaltkreis (20) eine Pulsberechnungseinheit (28) umfasst, die einen Pulswert (P1, P2, P3) berechnet, der jeweils dem Empfangssignal (SR1, SR2, SR3) entspricht, welches von einem Empfänger (R1, R2, R3) erzeugt wird, und eine Auswahleinheit (30), welche unter den von der Pulsberechnungseinheit (28) erhaltenen Pulswerten (P1, P2, P3) einen optimalen Pulswert (PO) bestimmt.

6. Pulsmesser (10) nach einem der Ansprüche 1 bis 4, wobei der elektronische Schaltkreis (20) eine Einheit (26) zum Berechnen eines virtuellen Signals (SV) umfasst, welches einer Addition der Empfangssignale (SR1, SR2, SR3) entspricht, die von jedem der Empfänger (R1, R2, R3) erzeugt werden, eine Pulsberechnungseinheit (28), welche einen Pulswert (P1, P2, P3, PV) berechnet, der jeweils jedem von einem Empfänger (R1, R2, R3) erzeugten Empfangssignal (SR1, SR2, SR3) und dem virtuellen Signal (SV) entspricht, und eine Auswahleinheit (30), die unter den von der Pulsberechnungseinheit (28) erhaltenen Pulswerten (P1, P2, P3, PV) einen optimalen Pulswert (PO) bestimmt.

7. Pulsmesser (10) nach Anspruch 5 oder 6, wobei der elektronische Schaltkreis (20) eine Einheit (32) zum Berechnen eines Zuverlässigkeitsindex (IF) der Messungen umfasst, der eine Funktion der von der Pulsberechnungseinheit (28) erhaltenen Pulswerte (P1, P2, P3, PV) ist.

8. Verfahren zum Steuern eines Pulsmessers (10) nach einem der vorstehenden Ansprüche, welches einen Messschritt umfasst, im Rahmen dessen jede Lichtquelle (E1, E2, E3) einen Lichtstrahl (FL) aussendet, und jeder Empfänger (R1, R2, R3) in Abhängigkeit von dem empfangenen Licht ein Empfangssignal (SR1, SR2, SR3) erzeugt, und einen Schritt der Berechnung des Pulses, im Rahmen dessen ausgehend von dem von jedem Empfänger (R1, R2, R3) während des Messschrittes erzeugten Empfangssignals (SR1, SR2, SR3) ein Pulswert (P1, P2, P3) berechnet wird, wobei auf den Schritt der Berechnung des Pulses (P1, P2, P3) ein Auswahlschritt folgt, im Rahmen dessen ein optimaler Pulswert (PO) zwischen den bei dem Schritt der Berechnung des Pulses erhaltenen Pulswerten (P1, P2, P3) ausgewählt wird.

9. Verfahren zum Steuern eines Pulsmessers (10) nach einem der Ansprüche 1 bis 7, welches einen Messschritt umfasst, im Rahmen dessen jede Lichtquelle (E1, E2, E3) einen Lichtstrahl (FL) aussendet und jeder Empfänger (R1, R2, R3) ein Empfangssignal (SR1, SR2, SR3) in Abhängigkeit von dem empfangenen Licht erzeugt, und einen Schritt der Berechnung des Pulses, im Rahmen dessen ausgehend von dem von jedem Empfänger (R1, R2, R3) während des Messschrittes erzeugten Empfangssignal (SR1, SR2, SR3) ein Pulswert (P1, P2, P3) berechnet wird, wobei zwischen dem Schritt der Messung und dem Schritt der Pulsrechnung ein Schritt der Berechnung eines virtuellen Signals (SV) liegt, der einer Addition der von jedem der Empfänger (R1, R2, R3) erzeugten Empfangssignale (SR1, SR2, SR3) entspricht, wobei während des Schrittes der Berechnung des Pulses ausgehend von dem virtuellen Signal (SV) ein Pulswert (PV) berechnet wird, und wobei während des Auswahlschrittes der Wert des optimalen Pulses (PO) unter den bei dem Schritt der Berechnung des Pulses erhalten Pulswerten (P1, P2, P3, P4) ausgewählt wird.

10. Steuerverfahren nach Anspruch 8 oder 9, wobei auf den Schritt der Berechnung des Pulses ein Schritt einer Berechnung eines Zuverlässigkeitsindex (IF) der Messungen folgt, im Rahmen dessen ein Vergleich zwischen den Pulswerten (P1, P2, P3, PV) durchgeführt wird, die bei dem Schritt der Berechnung des Pulses erhalten worden sind.

11. Steuerverfahren gemäss dem vorstehenden Anspruch, wobei auf den Schritt der Berechnung eines Zuverlässigkeitsindex (IF) der Messungen ein Schritt der Detektierung des Zustands der Positionierung des Gehäuses (14) folgt, im Rahmen dessen in Abhängigkeit von dem Zuverlässigkeitsindex (IF) festgelegt wird, ob der Pulsmesser (10) getragen wird, oder ob das Gehäuse (14) schlecht auf dem Handgelenk (12) positioniert ist.
